# EUROPÄISCHE PATENTSCHRIFT

(11) **EP 3 177 370 B1**
(45) Veröffentlichungstag und Bekanntmachung des Hinweises auf die Patenterteilung: **19.12.2018**
(21) Anmeldenummer: 15756821.3
(22) Anmeldetag: 07.08.2015
(51) Int. Cl.: A63B 22/00, A63B 22/18, A63B 26/00

(54) **VORRICHTUNG ZUR POSTUROGRAPHIE**
DEVICE FOR POSTUROGRAPHY
DISPOSITIF DE POSTUROGRAPHIE

(30) Priorität: 08.08.2014 AT 505542014
(43) Veröffentlichungstag der Anmeldung: 14.06.2017
(73) Patentinhaber: Sense Product GmbH, 1030 Wien (AT)
(72) Erfinder: KOGLER, Thomas, 1030 Wien (AT)
(74) Vertreter: Sonn & Partner Patentanwälte
(86) Internationale Anmeldenummer: PCT/AT2015/050192
(87) Internationale Veröffentlichungsnummer: WO 2016/019407

(56) Entgegenhaltungen:
- EP-A2- 0 761 266
- US-A- 5 409 226
- US-A- 5 921 899
- US-A1- 2011 111 935

## Beschreibung

Die Erfindung betrifft eine Vorrichtung zur Posturographie gemäß dem Oberbegriff von Anspruch 1.

Ein solches Posturographie-Trainingsgerät ist beispielsweise aus der AT 507 646 B1 bekannt. Das bekannte Gleichgewichtstrainingsgerät weist einen lose auf einer starren Basisplatte aufliegenden Schlauchring auf, der an der Unterseite einer starren Platte befestigt ist. Durch Einströmen komprimierter Luft in den vom Schlauchring, von der Platte und von der Basisplatte begrenzten Raum wird ein Überdruck erzeugt, wobei ein die Platte anhebendes Luftkissen ausgebildet wird.

Als Posturographie werden Verfahren bezeichnet, die für die Analyse der Körperhaltung benutzt werden. Bei der Posturographie werden insbesondere Körperschwankungen beim ruhenden Stand im Lot registriert. Im Stand der Technik ist es bereits bekannt, die Körperhaltung mit Kraftmessplatten zu analysieren. Hierbei kann die Lage von der Projektion des Körperschwerpunktes, der sogenannte Druckmittelpunkt, objektiv ermittelt werden. Wenn sich das Körpergewicht ungleichmäßig auf der Messplattform verteilt, wandert der Druckmittelpunkt je nach Verlagerung des Gesamtkörpergewichts nach vorne oder nach hinten, nach links oder nach rechts. Solche Abweichungen können graphisch an einem Computerbildschirm dargestellt werden. Diese Daten können zu verschiedenen diagnostischen Zwecken verwendet werden.

Bei umfangreichen theoretischen und praktischen Versuchen hat sich jedoch gezeigt, dass die bekannten Systeme mit Kraftmessplatten nicht in der Lage waren, die Koordinations- und Gleichgewichtsfähigkeiten der Testpersonen mit der gewünschten Präzision zu analysieren.

Die US 2008/0228110 A1 beschreibt eine andersartige Vorrichtung für die Posturografie, welche eine Plattform mit einer Kraftmessplatte aufweist, die an der Oberseite einer quadratischen oberen Platte befestigt wird. Darüber hinaus ist eine quadratische untere Platte vorgesehen, welche an Füßen befestigt ist. Am Außenumfang zwischen der oberen Platte und der unteren Platte sind nachgiebige Elemente angeordnet. Diese Elemente können Federn oder Dämpfer sein. Als Beispiel werden auch kompressible Schaumstoffelemente angegeben. Mangels Luftkissen ist bei dieser Vorrichtung jedoch eine Beweglichkeit in horizontaler Richtung nicht gegeben, so dass die Testergebnisse nicht zufriedenstellend sind. Darüber hinaus ist nachteilig, dass die Feder- bzw. Dämpfungselemente lediglich an den Eckpunkten zwischen der jeweils quadratischen oberen und unteren Platte vorgesehen sind. Dadurch ist das Verhalten der Vorrichtung stark von der Kipprichtung abhängig.

Die WO 2013/134873 A1 beschreibt eine andersartige Vorrichtung, bei welcher ein Patient auf einem nachgiebigen Material wie Schaumstoff stehen kann.Die US 5,409,226 beschreibt einen andersartigen Apparat zur Aufnahme von Positionssignalen einer Plattform, welche um die zwei rotatorischen Freiheitsgrade senkrecht zur Achse eines Schwenkbolzens drehbar gelagert ist. Der Schwenkbolzen ist dabei zwischen Dämpfern und Messwertwandlern angeordnet. Zwischen der Plattform und einer ringförmigen Basis ist eine pneumatische Feder angeordnet, welche durch einen mit konstanten Luftdruck gefüllten Schlauch gebildet wird und eine rückstellende Kraft auf die Plattform in Richtung der horizontale Ausgangslage der Plattform ausübt. Um einen Anschlag der Plattform zu verhindern, sind Anschlagspuffer vorgesehen. Mit Hilfe eines Luftzylinders lässt sich über einen Bolzen das Chassis der Anordnung in vertikale Richtung aktuieren sowie das Gewicht des Benutzers messen.

Die US 5,921,899 zeigt eine andersartige Trainingseinrichtung zur Simulation von Sprungbewegungen bei gleichzeitiger Haltemöglichkeit.

Die EP 0761266 offenbart eine andersartige Plattform für "Virtual Reality"-Anwendungen.

Die US 2011/0111935 betrifft ein andersartiges Trainingsgerät mit einer Plattform, die mit einer aufblasbaren Unterstützung verbunden ist.

Demgegenüber besteht die Aufgabe der vorliegenden Erfindung darin, eine Vorrichtung zur Posturographie der eingangs angeführten Art zu schaffen, mit welcher die Analyse der Körperhaltung mit konstruktiv einfachen Mitteln verbessert werden kann.

Diese Aufgabe wird durch eine Vorrichtung mit den Merkmalen von Anspruch 1 gelöst.

Erfindungsgemäß ist zwischen der Messplattform und dem Grundelement ein Dämpfungselement mit einem Dämpfungsmaterial angeordnet, mit welchem eine Kippbewegung der Messplattform gedämpft wird.

Die Erfindung beruht also darauf, dass die Kippbewegungen der Messplattform bei der Posturographie durch das Dämpfungsmaterial ständig gefedert bzw. gedämpft werden, so dass ein harter Anschlag der Messplattform in vertikaler Richtung während des Tests vermieden wird. Darüber hinaus kann mit der Luftkisseneinrichtung eine horizontale Beweglichkeit der Messplattform ermöglicht werden. Die Messplattform ist vorzugsweise um eine vertikale Achse durch den Mittelpunkt der Messplattform verschwenkbar gelagert. Vorteilhafterweise kann daher eine Beweglichkeit besonders vielen Freiheitsgraden erzielt werden. Es hat sich überraschend herausgestellt, dass mit der erfindungsgemäßen Vorrichtung die Aussagekraft der Messdaten wesentlich verbessert werden kann. Der Grund dafür ist, dass das Gleichgewicht der Testperson beim Testprogramm permanent, ohne Einstellung auf einen harten Endanschlag, dynamisch trainiert wird. Die Anordnung des Dämpfungselements trägt daher maßgeblich zur korrekten Messung bei. Würde die Messplattform hingegen auf einer starren Platte anschlagen, wäre eine korrekte Posturographie in einem Durchgang nicht möglich bzw. wäre die Messung fehlerhaft. Vorteilhafterweise können daher die Ergebnisse der Posturographie wesentlich verbessert werden. Das erfindungsgemäße Posturographie-Gerät ist insbesondere dafür geeignet, ein individuelles sensomotorisches Stärken- und Schwächen-Profil für die Testperson zu entwickeln. Darüber hinaus können wirksame Sturzpräventionsprogramme erstellt werden. Die erfindungsgemäße Vorrichtung kombiniert in vorteilhafterweise die translatorische Beweglichkeit der Messplattform durch die Luftkisseneinrichtung mit der gedämpften Verkippbarkeit der Messplattform durch die Kippeinrichtung und das Dämpfungselement. Dadurch wird eine maximale Destabilisierung der Testperson erzielt, welche eine besonders aufschlussreiche Analyse ermöglicht.

Um die Kippbewegung der Messplattform in alle Richtungen gleichmäßig zu dämpfen, ist es günstig, wenn das Dämpfungsmaterial umlaufend, insbesondere ringförmig umlaufend, zwischen der Messplattform und dem Grundelement angeordnet ist. Das Dämpfungsmaterial erstreckt sich daher vorzugsweise mit gleichbleibendem Querschnitt umlaufend zwischen der Messplattform und dem Grundelement. Bevorzugt erstreckt sich das Dämpfungsmaterial unterhalb eines radial äußeren Randbereiches der Messplattform. Bei der Posturographie kann die Messplattform durch die Testperson ständig aus der Mittelstellung in eine beliebige Richtung verkippt werden, wobei der darunterliegende Abschnitt des Dämpfungsmaterials entsprechend komprimiert wird. Dadurch kann die Kippbewegung der Messplattform für jeden Kippwinkel in demselben Ausmaß gedämpft werden. Vorteilhafterweise kann so ein harter Anschlag der Messplattform in vertikaler Richtung, d.h. in z-Richtung, zuverlässig vermieden werden, wodurch die Aussage- und Prognosekraft der Analyse wesentlich gesteigert werden kann.

Um die Kippbewegungen der Messplattform bei der Posturographie zu dämpfen bzw. zu federn, ist es günstig, wenn das Dämpfungsmaterial in einer im Wesentlichen waagrechten Mittelstellung der Messplattform an deren Unterseite anliegt. Demnach tritt die Dämpfungs- bzw. Federwirkung sofort ein, wenn die Messplattform aus der im Wesentlichen waagrechten Mittelstellung verkippt wird.

Zur Anpassung der Dämpfungscharakteristik ist es günstig, wenn das Dämpfungsmaterial im Querschnitt in Richtung der Messplattform hin verjüngt ist. Diese Ausführung hat den Vorteil, dass bei geringen Auslenkungen der Messplattform aus der Mittelstellung eine schwächere Dämpfungswirkung und damit eine stärkere Destabilisierung der Testperson als bei größeren Auslenkungen der Messplattform erzielt wird. Dadurch können die motorischen bzw. koordinativen Fähigkeiten der Testperson auf optimale Weise untersucht werden.

Um die gewünschten Dämpfungseigenschaften zu erzielen, kann einerseits die Querschnittsgeometrie des Dämpfungsmaterials und andererseits das Dämpfungsmaterial selbst angepasst werden. Je stärker das Dämpfungsmaterial zur Messplattform hin zusammenläuft, umso schwächer ist die Dämpfungswirkung, so dass ein entsprechend härteres Dämpfungsmaterial vorzusehen ist. Umgekehrt kann ein weicheres Dämpfungsmaterial verwendet werden, wenn die Querschnittsfläche des Dämpfungsmaterials vergrößert wird.

Gemäß einer besonders bevorzugten Ausführung weist das Dämpfungsmaterial im Querschnitt eine im Wesentlichen parabelförmige Begrenzungsfläche auf. Dadurch wird ein progressives Dämpfungsverhalten erhalten, welches sich für die Posturografie besonders gut eignet. Bevorzugt ist die in radialer Richtung gesehen äußere Begrenzungsfläche des Dämpfungsmaterials im Querschnitt im Wesentlichen parabelförmig. Vorteilhafterweise nimmt daher die Dämpfungswirkung des Dämpfungsmaterials mit steigendem Kippwinkel kontinuierlich zu. Entsprechend nimmt die Destabilisierung der Testperson ab, ohne jedoch zur Gänze zu verschwinden. Die im Wesentlichen parabelförmige Begrenzungsfläche ist dabei bevorzugt als Anlagefläche für die Unterseite der Messplattform ausgebildet.

Darüber hinaus ist es günstig, wenn das Dämpfungsmaterial eine erste Schicht auf Seite der Messplattform und eine zweite Schicht auf Seite des Grundelements aufweist, wobei die zweite Schicht härter als die erste Schicht ist. Demnach ist die erste Schicht, d.h. bezogen auf die Betriebsstellung die obere Schicht, des Dämpfungsmaterials weicher als die zweite bzw. untere Schicht des Dämpfungsmaterials. Diese Ausführung hat zur Folge, dass die Dämpfungswirkung mit dem Kippwinkel ausgehend von der Mittelstellung der Messplattform zunimmt. Demnach wird die Testperson bei kleineren Kippwinkeln stärker als bei größeren Kippwinkeln destabilisiert.

Zur Vermeidung eines harten Anschlages für die Verschwenk- bzw. Kippbewegung der Messplattform hat es sich als günstig erwiesen, wenn als Dämpfungsmaterial ein Schaumstoff, insbesondere aus Polyurethan, vorgesehen ist. Durch den Schaumstoff bleibt die Testperson auf der Messplattform ständig in Bewegung, wodurch eine besonders vorteilhafte unterbrechungsfreie Posturographie ermöglicht wird. Das Dämpfungsmaterial zwischen der Messplattform und dem Grundelement ist derart ausgebildet und angeordnet, dass bei der Posturographie Kippwinkel von zwischen 5 und 20 Grad, vorzugsweise im Wesentlichen 10 Grad, jeweils bezogen auf die waagrechte Mittelstellung der Messplattform, erzielbar sind. Demnach kann die Mittelachse der Messplattform um einen maximalen Kippwinkel von 5 bis 20 Grad, insbesondere im Wesentlichen 10 Grad, aus ihrer vertikalen Normalstellung in alle Richtungen gekippt werden. Die Kippbewegung der Messplattform wird bis zum Erreichen des maximalen Kippwinkels durch das Dämpfungsmaterial gedämpft. Bevorzugt beträgt der maximale Kippwinkel im Wesentlichen 10 Grad.

Zur Erzielung einer Modulbauweise ist es günstig, wenn das Dämpfungselement über eine lösbare Verbindung, insbesondere eine Steckverbindung, mit dem Grundelement und/oder dass die Messplattform über eine weitere lösbare Verbindung, insbesondere eine weitere Steckverbindung, mit dem Dämpfungselement verbunden ist. Vorteilhafterweise können so die einzelnen Module einfach, insbesondere werkzeuglos, ausgetauscht werden. Dies ist insbesondere für das Dämpfungselement günstig, bei welchem ein anderes Dämpfungsmaterial für unterschiedliche Einsatzzwecke ausgewählt werden kann.

Um das Dämpfungselement mit konstruktiv einfachen Mitteln in der bestimmungsgemäßen Position auf dem Grundelement anzuordnen, ist es vorteilhaft, wenn das Dämpfungselement zumindest eine Zentrieröffnung zur Aufnahme eines entsprechenden Zentrierelementes des Grundelementes aufweist. Alternativ kann das Grundelement eine Zentrieröffnung aufweisen, in welcher ein entsprechendes Zentrierelement des Dämpfungselementes aufnehmbar ist.

Um die Posturographie wahlweise mit und ohne Verkippungen durchzuführen, ist es günstig, wenn zwischen dem Grundelement oder dem Dämpfungselement und der Messplattform zumindest drei Kipphebelelemente vorgesehen sind, welche zwischen einer die Kippbewegung der Messplattform freigebenden Freigabestellung und einer die Kippbewegung der Messplattform sperrenden Sperrstellung verschwenkbar sind. Bevorzugt sind die Kipphebelelemente gelenkig am Grundelement gelagert. Durch Verschwenken in die Sperrstellung werden die Kipphebelelemente mit der Messplattform in Anschlag gebracht. Alternativ können die Kipphebelelemente auch an der Messplattform gelenkig angebracht sein. Auf diese Weise kann die Posturographie-Vorrichtung für verschiedene Anwendungen verwendet werden. Durch manuelles Verschwenken der Kipphebelelemente in die Sperrstellung wird die Kippbewegung der Messplattform blockiert, so dass die Posturographie auf die horizontale Bewegung, d.h. die Bewegung in x-y-Richtung, der Messplattform beschränkt ist. Um die Verschwenkbarkeit der Messplattform freizugeben, werden die Kipphebelelemente in die Freigabestellung verschwenkt.

Um die Kipphebelelemente zuverlässig in der Sperrstellung zu halten, ist es vorteilhaft, wenn an den Kipphebelelementen Rastelemente zur Verrastung in der Sperrstellung vorgesehen sind. Beim Verschwenken der Kipphebelelemente nach innen wird die Rastverbindung hergestellt. Als Rastelemente sind bevorzugt Rastnasen vorgesehen, welche mit entsprechenden Rastaufnahmen insbesondere an der Messplattform zusammenwirken. Dadurch kann einerseits verhindert werden, dass die Messplattform versehentlich zwischen den beiden Betriebsstellungen, mit oder ohne Kippmöglichkeit, verstellt wird. Andererseits wird die Umstellung für den Benutzer besonders einfach gestaltet.

Um die Gewichtsverlagerungen des Benutzers auf der Messplattform präzise zu erfassen, ist es von Vorteil, wenn die Messplattform eine Vielzahl von vorzugsweise im Wesentlichen regelmäßig an der Aufstandsfläche angeordneten Drucksensoren aufweist. Damit kann ein Belastungsprofil erstellt werden. Besonders günstig ist es, wenn an der Aufstandsfläche zwischen 4000 und 6600, vorzugsweise zwischen 4700 und 5900, insbesondere ungefähr 5300 Sensoren vorgesehen sind. Solche Drucksensoren sind im Stand der Technik an sich bekannt. Die Signale der Drucksensoren werden zur Auswertung an eine Recheneinrichtung übertragen.

Für die Posturographie ist es günstig, wenn die Messplattform zur Ausbildung der Aufstandsfläche eine kreisförmige Sensorfolie mit den Drucksensoren aufweist, welche bevorzugt aus mehreren Sensorfolienteilen, insbesondere aus vier im Wesentlichen viertelkreisförmigen Sensorfolienteilen, zusammengesetzt ist. Derartige Sensorfolien mit Drucksensoren sind aus anderen Anwendungsgebieten an sich bekannt. Zur Verwendung bei der erfindungsgemäßen Vorrichtung zur Posturographie hat es sich jedoch als besonders günstig erwiesen, wenn die kreisförmige Sensorfolie aus vier viertelkreisförmigen Folien zusammengesetzt ist, welche im Wesentlichen spaltfrei nebeneinander an der Aufstandsfläche angeordnet sind.

Zur Verarbeitung der Messsignale ist es vorteilhaft, wenn die Drucksensoren der Sensorfolienteile mit Signalabnehmern verbunden sind, welche über Datentransferleitungen mit einer Recheneinrichtung verbunden sind. Vorzugsweise ist pro Sensorfolienteil genau ein Signalabnehmer vorgesehen, welcher die Messsignale der Drucksensoren an dem jeweiligen Sensorfolienteil aufnimmt. Die Messdaten werden über geeignete Datentransferleitungen an eine Recheneinrichtung übergeben, mit welcher eine Analyse der Messdaten vorgenommen wird.

Zur Ausbildung des Luftkissens ist es günstig, wenn die Luftkisseneinrichtung ein umlaufendes, einen Überdruckraum begrenzendes Dichtungselement aufweist. Der Überdruckraum wird durch das Dichtungselement, das Grundelement und eine Gleitfläche für das Dichtungselement begrenzt. Wie im Stand der Technik an sich bekannt, ist der Überdruckraum über eine Leitung mit einer Drucklufteinrichtung verbunden, mit welcher Druckluft in den Überdruckraum eingeleitet werden kann. Dadurch wird an der Unterseite des Grundelementes ein Luftkissen ausgebildet, mit welchem die horizontale Beweglichkeit der Messplattform bewirkt wird. Als Dichtungselement kann ein Schlauchring vorgesehen sein. Die Ausgestaltung der Luftkisseneinrichtung ist beispielsweise aus der AT 509 939 A1 bekannt, auf welche hiermit der Einfachheit halber verwiesen werden kann.

Wie bereits aus der AT 509 939 A1 bekannt geworden ist, kann das Grundelement in einem Rahmenelement in horizontaler Ebene beweglich angeordnet sein. Hinsichtlich der Lagerung des Grundelements an dem Rahmen- bzw. Basiselement kann daher wiederum auf die AT 509 939 A1 verwiesen werden.

Um bei der Posturographie einen harten Anschlag für Bewegungen der Messplattform in horizontaler Ebene zu vermeiden, ist es günstig, wenn das Rahmenelement ein Aufnahmeelement für ein Führungselement des Grundelementes aufweist, wobei zwischen dem Führungselement und dem Aufnahmeelement ein Dämpfungsanschlag ausgebildet ist. Der Dämpfungsanschlag weist ein Dämpfungsmaterial, beispielsweise Schaumstoff oder Moosgummi, auf, mit welchem der Anschlag des Führungselementes des Grundelementes an dem Aufnahmeelement gedämpft wird. Bevorzugt ist das Dämpfungsmaterial ringförmig an der Innenseite des Aufnahmeelementes vorgesehen. Vorteilhafterweise können so die Bewegungen der Messplattform in alle drei Raumrichtungen gedämpft durchgeführt werden.

In der Praxis hat es sich beim Stand der Technik als problematisch erwiesen, dass der Schlauchring der Luftkisseneinrichtung durch Gewichtsverlagerungen auf der Messplattform übermäßig verformt wird. Aus diesem Grund ist es vorteilhaft, wenn zwischen dem Grundelement und dem Rahmenelement ein Distanzelement vorgesehen ist. Das Distanzelement ist dazu eingerichtet, den Abstand zwischen der Gleitfläche des Dichtungselementes der Luftkisseneinrichtung und dem Grundelement bei der Posturographie im Wesentlichen konstant zu halten. Bevorzugt sind mehrere Distanzelemente radial außerhalb des Dichtungselementes vorgesehen, um einer vertikalen Verformung des Dichtungselementes bei Belastungen nach allen Seiten hin entgegenwirken.

Bei dieser Ausführung ist es günstig, wenn das Distanzelement ein in vertikaler Richtung elastisch verformbares Federelement aufweist. Die Federelemente weisen bevorzugt jeweils zumindest einen Federbügel auf, welcher an der Unterseite des Grundelementes befestigt ist.

Darüber hinaus ist es günstig, wenn das Distanzelement ein Gleitelement zum Gleiten auf einer Gleitfläche des Rahmenelementes aufweist. Als Gleitelement kann insbesondere ein Kugelelement vorgesehen sein, welches am freien Ende des Federelementes angeordnet ist. Bevorzugt ist jedes Gleitelement über zwei Federbügel mit dem Grundelement verbunden.

Die Erfindung wird nachstehend anhand eines bevorzugten Ausführungsbeispiels, auf das sie jedoch nicht beschränkt sein soll, noch weiter erläutert. In der Zeichnung zeigen
Fig. 1 eine Schnittansicht einer erfindungsgemäßen Vorrichtung zur Posturographie in Modulbauweise mit einer Messplattform, welche an einem Grundelement kippbar gelagert ist, wobei zwischen der Messplattform und dem Grundelement ein die Kippbewegungen der Messplattform dämpfendes Dämpfungselement angeordnet ist;
Fig. 2 eine schaubildliche Ansicht der Messplattform der in Fig. 1 dargestellten Vorrichtung;
Fig. 3 eine schaubildliche Ansicht des Dämpfungselements der in Fig. 1 dargestellten Vorrichtung;
Fig. 4 eine schaubildliche Ansicht des Grundelements der in Fig. 1 dargestellten Vorrichtung;
Fig. 5 eine Seitenansicht, Fig. 6 eine Draufsicht und Fig. 7 eine Vorderansicht eines Diagnose- und Therapiegeräts, bei welchem die Vorrichtung gemäß Fig. 1 bis 4 eingesetzt wird; und
Fig. 8 eine Schnittansicht von Teilen einer weiteren erfindungsgemäßen Vorrichtung zur Posturographie, bei welcher das Dämpfungselement eine parabelförmige Begrenzungsfläche und einen zweischichtigen Aufbau aufweist.

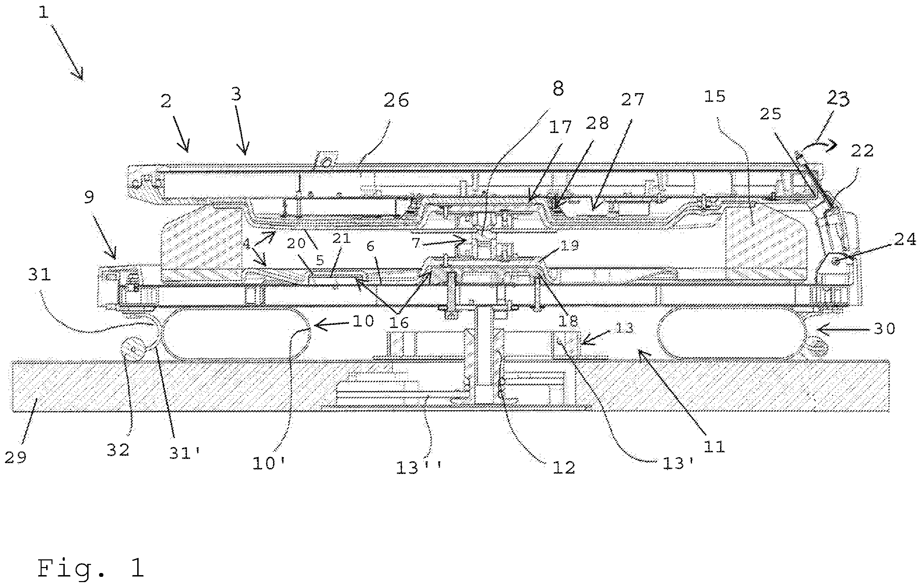

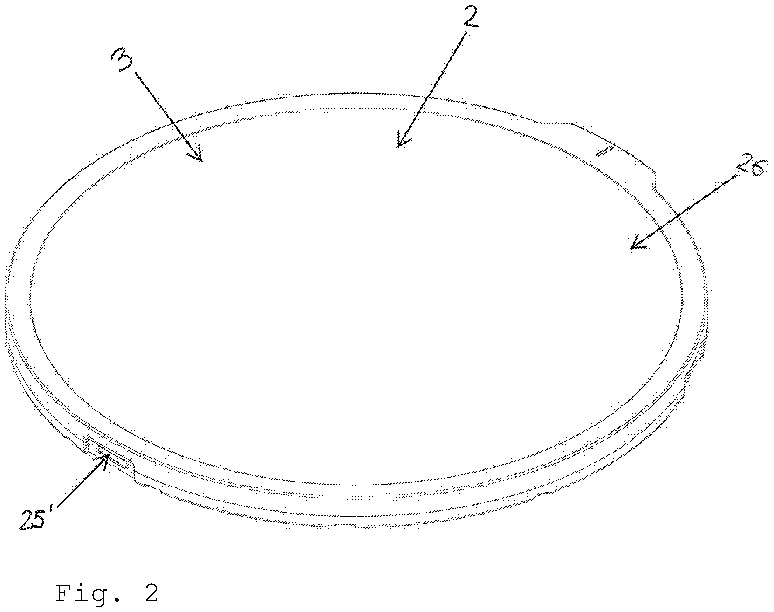

Fig. 1 zeigt eine Vorrichtung 1 zur Posturographie, mit welchem eine Analyse der Körperhaltung eines Benutzers vorgenommen werden kann. Das Trainingsgerät 1 weist eine Messplattform 2 auf, welche in Fig. 2 dargestellt ist. An der Oberseite der Messplattform 2 ist eine Aufstandsfläche 3 vorgesehen, auf welcher die zu untersuchende Person bei der Posturographie steht.

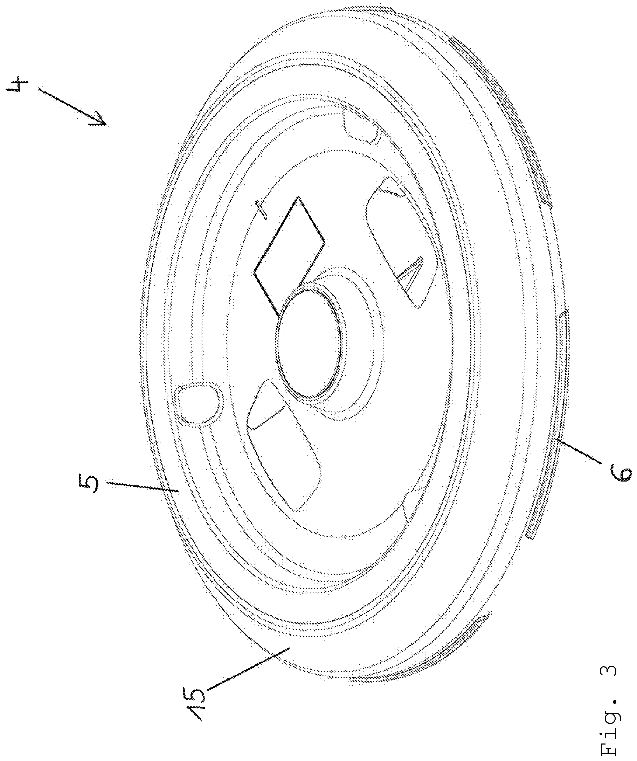

Wie aus Fig. 1 weiters ersichtlich, weist das Trainingsgerät 1 zudem ein Dämpfungselement 4 (vgl. Fig. 3) auf, welches auf der Oberseite eine Kippplatte 5 und an der Unterseite eine Lagerplatte 6 aufweist. Die Messplattform 2 ist zusammen mit der Kippplatte 5 des Dämpfungselementes 4 mittels einer Kippeinrichtung 7 kippbar relativ zur Lagerplatte 6 des Dämpfungselementes 4 gelagert. Die Kippeinrichtung 7 weist in der gezeigten Ausführung ein Gelenk 8, in der gezeigten Ausführung ein Kreuzgelenk, zwischen der Kippplatte 5 und der Lagerplatte 6 auf. Das Kreuzgelenk 8 ist entlang einer zentralen Achse der Messplattform 2 angeordnet. Die Messplattform 2 kann mittels des Kippgelenks 8 bei der Posturographie in alle Richtungen um die zentrale Achse kippen.

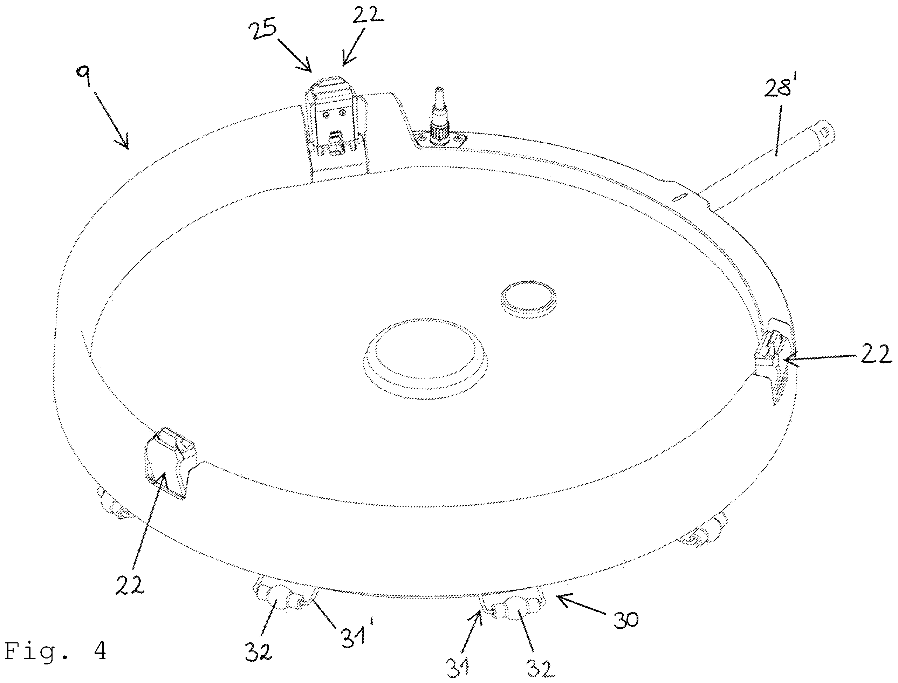

Wie aus Fig. 1 weiters ersichtlich, weist das Trainingsgerät 1 zudem ein Grundelement 9 (vgl. Fig. 4) auf, welches an der Unterseite mit einer Luftkisseneinrichtung 10 verbunden ist. Mit Hilfe der Luftkisseneinrichtung 10 kann bei der Posturographie ein das Grundelement 4 tragendes Luftkissen ausgebildet werden. Die Luftkisseneinrichtung 10 weist ein ringförmig umlaufendes Dichtungselement 10' in Form eines Schlauchrings oder eines Ringkörpers auf. Das Dichtungselement 10 begrenzt einen Überdruckraum 11 unterhalb des Grundelementes 4, welcher mit einer (nicht gezeigten) Luftdruckeinrichtung verbunden ist. Aufgrund des Luftkissens kann die Messplattform 2 bei der Posturographie in horizontaler Ebene in alle Richtungen im Wesentlichen frei bewegt werden. Darüber hinaus ist das Grundelement 9 an der Unterseite mit einem rohrförmigen Führungselement 12 verbunden, welches innerhalb eines Aufnahmeelementes 13 beweglich ist. Dadurch kann die maximale horizontale Beweglichkeit der Messplattform 2 begrenzt werden. Das Aufnahmeelement 13 weist an der Innenseite einen Dämpfungsanschlag 13' aus einem elastisch verformbaren Dämpfungsmaterial, beispielsweise Schaumstoff, auf. Damit kann ein harter Anschlag des Führungselementes 12 vermieden werden, welcher die Aussagekraft der gesammelten Bewegungsdaten beeinträchtigen würde. In der gezeigten Ausführung ist der Dämpfungsanschlag 13' ringförmig ausgebildet. Darüber hinaus ist in Fig 1 ein Rückstellelement 13" in Form eines elastischen Seiles ersichtlich, mit welchem das Führungselement 12 in Richtung einer Mittelstellung innerhalb des Aufnahmeelementes 13 rückgestellt wird. Die elastischen Seile haben die Aufgabe, den gesamten Innenteil zu zentrieren, so dass stets das gleiche Ausgangsbild bei der Messung gewährleistet wird.

Wie aus Fig. 1 weiters ersichtlich, weist das Feder- und Dämpfungselement 4 zwischen der Messplattform 2 und dem Grundelement 9 ein Dämpfungsmaterial 15 auf, mit welchem jegliche Kippbewegungen der Messplattform 2 gedämpft werden. Aufgrund des Dämpfungsmaterials 15 wird daher eine Verschwenkung der Messplattform 2 bezüglich deren Haupterstreckungsebene sanft gebremst. Das Dämpfungsmaterial 15 erstreckt sich in der gezeigten Ausführung ringförmig umlaufend unterhalb der Messplattform 2. In Fig. 4 ist die Messplattform 2 in einer im Wesentlichen waagrechten Mittelstellung gezeigt, aus welcher die Messplattform 2 bei der Posturographie in alle Richtungen gekippt werden kann. In der gezeigten Mittelstellung ist das Dämpfungsmaterial 15 an der Unterseite der Kippplatte 5 bzw. der Messplattform 2 angelegt, so dass die Dämpfungswirkung beim Verkippen aus der Mittelstellung sofort eintritt. Für die Zwecke dieser Offenbarung beziehen sich die Richtungsangaben wie "oben", "unten" etc. auf die gezeigte Betriebsstellung der Vorrichtung 1.

Wie aus Fig. 1 weiters ersichtlich, ist das Dämpfungsmaterial 15 in der gezeigten Ausführung im Querschnitt in Richtung der Messplattform 2, d.h. nach oben hin, verjüngt ausgebildet. Zur Anpassung der Dämpfungscharakteristik können unterschiedliche Querschnittgeometrien des Dämpfungsmaterials 15 vorgesehen sein.

Als Dämpfungsmaterial 15 ist in der gezeigten Ausführung ein offenporiger Schaumstoff vorgesehen, mit welchem die gewünschte Dämpfung erzielt wird, aber zugleich der für die Posturographie erforderliche maximale Kippwinkel von circa 10 Grad bezüglich der horizontalen Ruhestellung gewährleistet wird. Bei der Posturographie kann die Messplattform permanent um bis zu 10 Grad in alle Richtungen verkippen, wobei die Testperson durch die Dämpfung stets in Bewegung gehalten wird.

Wie aus Fig. 1 ersichtlich, weist die Vorrichtung 1 zur Posturographie eine Modulbauweise auf, wobei einerseits das Dämpfungselement 4 über eine werkzeuglos lösbare Verbindung 16 mit dem Grundelement 9 und andererseits die Messplattform 2 über eine weitere werkzeuglos lösbare Verbindung 17 mit dem Dämpfungselement 4 verbunden ist. In der gezeigten Ausführung kann das Dämpfungselement 4 in der bestimmungsgemäßen Lage auf das Grundelement 9 aufgesetzt werden. Zu diesem Zweck weist das Grundelement 9 eine zentrale Erhebung 18 auf, in welche eine entsprechende Vertiefung 19 des Dämpfungselementes 4 passt. Darüber hinaus weist das Dämpfungselement 4 eine Zentrierausnehmung 20 zur Aufnahme eines entsprechenden Zentrierelementes 21 des Grundelementes 9 auf.

Wie aus Fig. 1 weiters ersichtlich, sind zwischen dem Grundelement 9 und der Messplattform 2 zumindest drei Kipphebelelemente 22 vorgesehen, welche in Pfeilrichtung 23 zwischen einer die Kippbewegung der Messplattform freigebenden Freigabestellung (nicht gezeigt) und einer die Kippbewegung der Messplattform 2 sperrenden Sperrstellung (vgl. Fig. 1) verschwenkbar sind. Die Kipphebelelemente 22 sind in der gezeigten Ausführung über Gelenke 24 am Grundelement 9 verschwenkbar gelagert. An den Kipphebelelementen 22 sind jeweils Rastelemente 25 in Form von Rastnasen vorgesehen, mit welchen die Kipphebelelemente 22 in der Sperrstellung mit entsprechenden Rastmitteln 25' an der Messplattform 2 (vgl. Fig. 2) verrastbar sind.

Wie aus Fig. 1 weiters ersichtlich, weist die Messplattform 2 zur Ausbildung der Aufstandsfläche 3 eine kreisförmige Sensorfolie 26 auf, welche mit einer Vielzahl von Drucksensoren ausgebildet ist. Die Sensorfolie 26 ist unterhalb einer Deckfolie angeordnet, mit welcher die Sensorfolie 26 gegenüber Scherkräften geschützt wird. Die Drucksensoren sind regelmäßig über die Aufstandsfläche 3 verteilt, um Gleichgewichtsverlagerungen der Testperson präzise zu erfassen. In der gezeigten Ausführung ist die Sensorfolie 26 aus vier im Wesentlichen viertelkreisförmigen Sensorfolienteilen zusammengesetzt. Die Drucksensoren der einzelnen Sensorfolienteile sind jeweils mit einem Signalabnehmer 27 verbunden, welcher über Datentransferleitungen 28 und 28' (vgl. Fig. 4) mit einer Recheneinrichtung (nicht gezeigt) verbunden sind.

Wie aus Fig. 1 weiters ersichtlich, ist das Grundelement 9 in einem schematisch gezeigten Rahmenelement 29 mit dem Aufnahmeelement 13 in horizontaler Ebene beweglich angeordnet. Zwischen dem Grundelement 9 und dem Rahmenelement 2 sind Distanzelemente 30 vorgesehen (vgl. auch Fig. 4). Die Distanzelemente 30 weisen jeweils ein in vertikaler Richtung elastisch verformbares Federelement 31 auf, welches in der gezeigten Ausführung durch zwei Bogenfedern 31' gebildet ist. Die Distanzelemente 30 weisen auf Seite des Rahmenelementes 20 jeweils ein kugelförmiges Gleitelement 32 zum Gleiten auf einer Gleitfläche 33 des Rahmenelementes 29 auf.

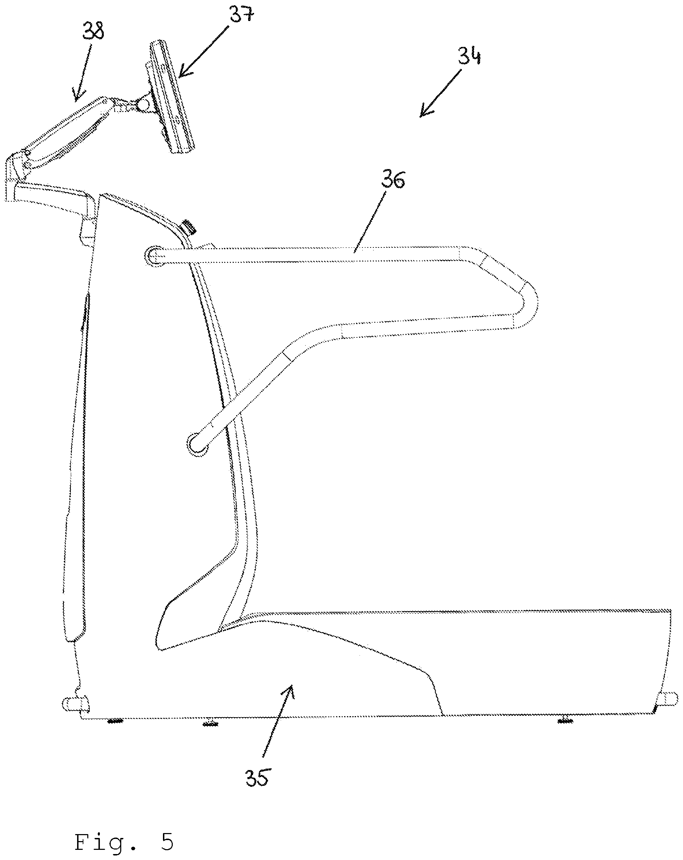

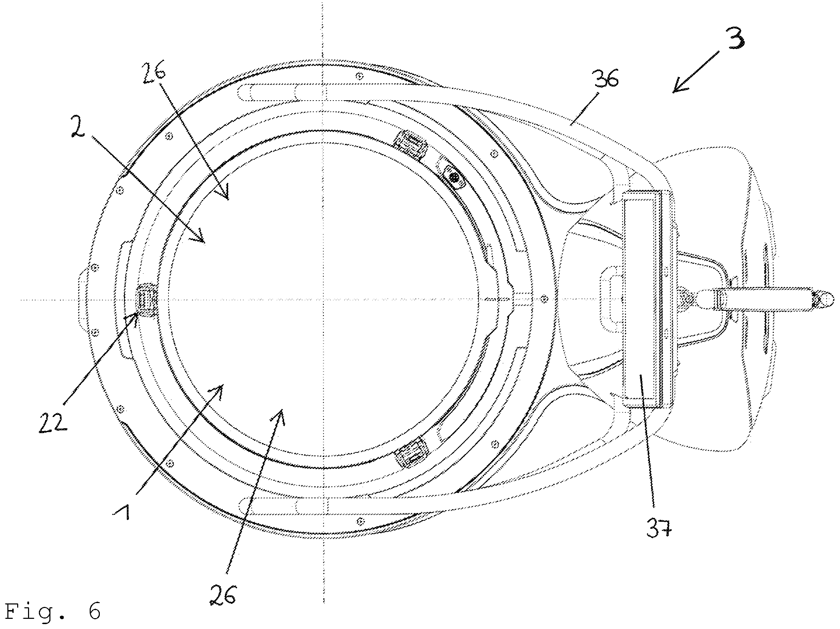

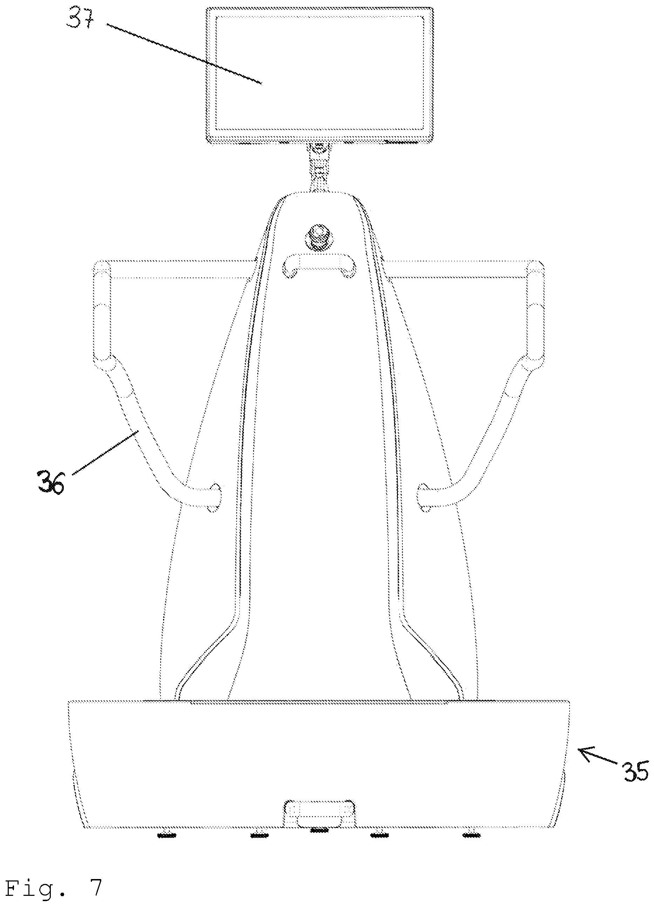

Fig. 5 bis 7 zeigt ein Diagnose- und Therapiegerät 34, bei welchem die Vorrichtung 1 gemäß den Fig. 1 bis 4 eingesetzt wird. Das Diagnose- und Therapiegerät 34 weist einen Rahmenaufbau 35 mit dem Rahmenelement 29 auf, in welchem das Aufnahmeelement 13 für das Innenteil bestehend aus der Messplattform 2, dem Dämpfungselement 4 und dem Grundelement 9 ausgebildet ist. Weiters ist ein Handlauf 36 ersichtlich, an welchem sich die Testperson anhalten kann. Zudem ist ein Bildschirm 37 zur Anzeige von Informationen gezeigt, welcher an einem Haltearm 38 verschwenkbar gelagert ist.

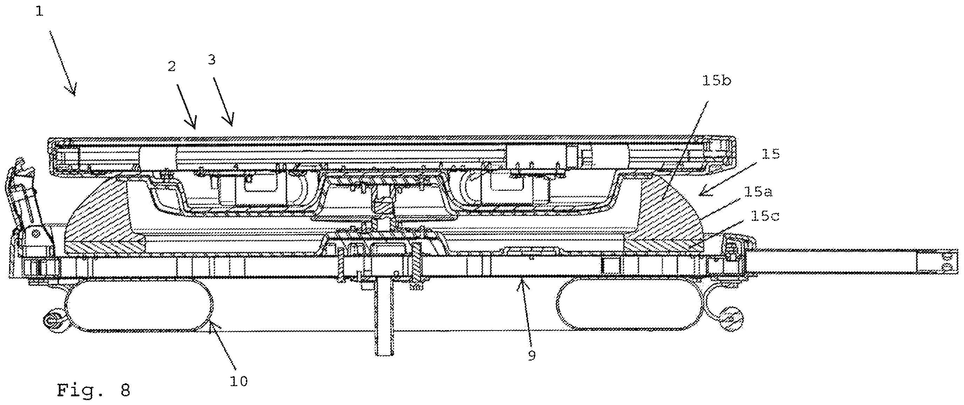

Fig. 8 zeigt eine alternative Ausführung der Vorrichtung 1 zur Posturographie, wobei nachstehend lediglich auf die Unterschiede zur vorangehenden Ausführungsform einzugehen ist.

Gemäß Fig. 8 weist das Dämpfungsmaterial 15 im Querschnitt (d.h. in einer Schnittebene senkrecht zur Hauptebene der Messplattform in der Mittelstellung) eine im Wesentlichen parabelförmige Begrenzungsfläche 15a auf. Demnach ist die Außenseite des Dämpfungselementes 4, an der die Messplattform 4 anliegt, parabelförmig gekrümmt. Darüber hinaus weist das Dämpfungsmaterial 15 in der gezeigten Ausführung eine erste Schicht 15b auf Seite der Messplattform 2 und eine zweite Schicht 15c auf Seite des Grundelements 9 auf. Die zweite Schicht 15c ist härter als die erste Schicht 15b, so dass die zweite Schicht 15c einen größeren Verformungswiderstand als die erste Schicht 15b aufweist. Beide Maßnahmen bewirken, dass das Dämpfungsverhalten des Dämpfungselements 4 bezüglich des Kippwinkels progressiv ist. Dadurch nimmt die Destabilisierung der Testperson mit dem Kippwinkel ab.

## Patentansprüche

1. Vorrichtung (1) zur Posturographie mit einer Messplattform (2), welche eine Aufstandsfläche (3) für eine Testperson aufweist, mit einem Grundelement (9), an welchem die Messplattform (2) mittels einer Kippeinrichtung (7) kippbar gelagert ist, und mit einer Luftkisseneinrichtung (10), mit welcher ein das Grundelement (9) tragendes Luftkissen ausgebildet wird und welche eine horizontale Beweglichkeit der Messplattform (2) bewirkt, **dadurch gekennzeichnet, dass** zwischen der Messplattform (2) und dem Grundelement (9) ein Dämpfungselement (4) mit einem Dämpfungsmaterial (15) angeordnet ist, mit welchem eine Kippbewegung der Messplattform (2) gedämpft und gefedert wird.

2. Vorrichtung (1) zur Posturographie nach Anspruch 1, **dadurch gekennzeichnet, dass** das Dämpfungsmaterial (15) umlaufend, insbesondere ringförmig umlaufend, zwischen der Messplattform (2) und dem Grundelement (9) angeordnet ist.

3. Vorrichtung (1) zur Posturographie nach Anspruch 1 oder 2, **dadurch gekennzeichnet, dass** das Dämpfungsmaterial (15) in einer im Wesentlichen waagrechten Mittelstellung der Messplattform (2) an deren Unterseite anliegt.

4. Vorrichtung (1) zur Posturographie nach einem der Ansprüche 1 bis 3, **dadurch gekennzeichnet, dass** das Dämpfungsmaterial (15) im Querschnitt in Richtung der Messplattform (2) hin verjüngt ist, wobei das Dämpfungsmaterial (15) vorzugsweise im Querschnitt eine im Wesentlichen parabelförmige Begrenzungsfläche (15a) aufweist.

5. Vorrichtung (1) zur Posturographie nach einem der Ansprüche 1 bis 4, **dadurch gekennzeichnet, dass** das Dämpfungsmaterial (15) eine erste Schicht (15b) auf Seite der Messplattform (2) und eine zweite Schicht (15c) auf Seite des Grundelements (9) aufweist, wobei die zweite Schicht (15c) härter als die erste Schicht (15b) ist.

6. Vorrichtung (1) zur Posturographie nach einem der Ansprüche 1 bis 5, **dadurch gekennzeichnet, dass** als Dämpfungsmaterial (15) ein Schaumstoff, insbesondere aus Polyurethan, vorgesehen ist.

7. Vorrichtung (1) zur Posturographie nach einem der Ansprüche 1 bis 6, **dadurch gekennzeichnet, dass** das Dämpfungselement (15) über eine lösbare Verbindung (16), insbesondere eine Steckverbindung, mit dem Grundelement (9) und/oder dass die Messplattform (2) über eine weitere lösbare Verbindung (17), insbesondere eine weitere Steckverbindung, mit dem Dämpfungselement (4) verbunden ist.

8. Vorrichtung (1) zur Posturographie nach einem der Ansprüche 1 bis 7, **dadurch gekennzeichnet, dass** das Dämpfungselement (4) zumindest eine Zentrieröffnung (20) zur Aufnahme eines entsprechenden Zentrierelementes (21) des Grundelementes (9) aufweist.

9. Vorrichtung (1) zur Posturographie nach einem der Ansprüche 1 bis 8, **dadurch gekennzeichnet, dass** zwischen dem Grundelement (9) oder dem Dämpfungselement (4) und der Messplattform (2) zumindest drei Kipphebelelemente (22) vorgesehen sind, welche zwischen einer die Kippbewegung der Messplattform (2) freigebenden Freigabestellung und einer die Kippbewegung der Messplattform (2) sperrenden Sperrstellung verschwenkbar sind, wobei an den Kipphebelelementen (22) bevorzugt Rastelemente (25) zur Verrastung in der Sperrstellung vorgesehen sind.

10. Vorrichtung (1) zur Posturographie nach einem der Ansprüche 1 bis 9, **dadurch gekennzeichnet, dass** die Messplattform (2) eine Vielzahl von vorzugsweise im Wesentlichen regelmäßig an der Aufstandsfläche angeordnete Drucksensoren aufweist, wobei die Messplattform (2) zur Ausbildung der Aufstandsfläche (3) bevorzugt eine kreisförmige Sensorfolie (26) mit den Drucksensoren aufweist, welche bevorzugt aus mehreren Sensorfolienteilen, insbesondere aus vier im Wesentlichen viertelkreisförmigen Sensorfolienteilen, zusammengesetzt ist, wobei die Drucksensoren der Sensorfolienteile bevorzugt mit Signalabnehmern (27) verbunden sind, welche über Datentransferleitungen (28) mit einer Recheneinrichtung verbunden sind.

11. Vorrichtung (1) zur Posturographie nach einem der Ansprüche 1 bis 10, **dadurch gekennzeichnet, dass** die Luftkisseneinrichtung (10) ein umlaufendes, einen Überdruckraum (11) begrenzendes Dichtungselement (10') aufweist.

12. Vorrichtung (1) zur Posturographie nach einem der Ansprüche 1 bis 11, **dadurch gekennzeichnet, dass** das Grundelement (9) in einem Rahmenelement (29) in horizontaler Ebene beweglich angeordnet ist, wobei das Rahmenelement (29) bevorzugt ein Aufnahmeelement (13) für ein Führungselement (12) des Grundelementes (9) aufweist, wobei zwischen dem Führungselement (12) und dem Aufnahmeelement (13) ein Dämpfungsanschlag (13') ausgebildet ist.

13. Vorrichtung (1) zur Posturographie nach Anspruch 12, **dadurch gekennzeichnet, dass** zwischen dem Grundelement (9) und dem Rahmenelement (29) ein Distanzelement (30) vorgesehen ist.

14. Vorrichtung (1) zur Posturographie nach Anspruch 13, **dadurch gekennzeichnet, dass** das Distanzelement (30) ein in vertikaler Richtung elastisch verformbares Federelement (31) aufweist.

15. Vorrichtung (1) zur Posturographie nach Anspruch 13 oder 14, **dadurch gekennzeichnet, dass** das Distanzelement (30) ein Gleitelement (32) zum Gleiten auf einer Gleitfläche (33) des Rahmenelementes (29) aufweist.

## Claims

1. Posturography apparatus (1) comprising a measuring platform (2), which has a standing surface (3) for a test subject, comprising a base element (9), on which the measuring platform (2) is tiltably mounted by means of a tilt device (7), and comprising an air cushion device (10), by means of which an air cushion supporting the base element (9) is formed and which permits horizontal movement of the measuring platform (2), **characterised in that** a damping element (4) comprising a damping material (15) is arranged between the measuring platform (2) and the base element (9), by means of which damping element a tilting movement of the measuring platform (2) is damped and spring-loaded.

2. Posturography apparatus (1) according to claim 1, **characterised in that** the damping material (15) is arranged peripherally, in particular annularly peripherally, between the measuring platform (2) and the base element (9).

3. Posturography apparatus (1) according to either claim 1 or claim 2, **characterised in that** the damping material (15) rests on the underside of the measuring platform (2) in a substantially horizontal central position.

4. Posturography apparatus (1) according to any of claims 1 to 3, **characterised in that** the cross section of the damping material (15) tapers towards the measuring platform (2), the damping material (15) preferably having a substantially parabolic boundary surface (15a) in cross section.

5. Posturography apparatus (1) according to any of claims 1 to 4, **characterised in that** the damping material (15) comprises a first layer (15b) on the measuring platform (2) side and a second layer (15c) on the base element (9) side, the second layer (15c) being harder than the first layer (15b).

6. Posturography apparatus (1) according to any of claims 1 to 5, **characterised in that** a foam material, in particular made of polyurethane, is provided as the damping material (15).

7. Posturography apparatus (1) according to any of claims 1 to 6, **characterised in that** the damping element (15) is connected to the base element (9) via a detachable connection (16), in particular a plug-in connection, and/or **in that** the measuring platform (2) is connected to the damping element (4) via another detachable connection (17), in particular another plug-in connection.

8. Posturography apparatus (1) according to any of claims 1 to 7, **characterised in that** the damping element (4) comprises at least one centring opening (20) for receiving a corresponding centring element (21) of the base element (9).

9. Posturography apparatus (1) according to any of claims 1 to 8, **characterised in that** at least three rocker arm elements (22) are provided between the base element (9) or the damping element (4) and the measuring platform (2), which rocker arm elements can be pivoted between a release position that releases the tilting movement of the measuring platform (2) and a blocking position that blocks the tilting movement of the measuring platform (2), locking elements (25) for locking in the blocking position preferably being provided on the rocker arm elements (22).

10. Posturography apparatus (1) according to any of claims 1 to 9, **characterised in that** the measuring platform (2) comprises a plurality of pressure sensors preferably arranged at substantially regular intervals on the standing surface, the measuring platform (2) preferably comprising a circular sensor film (26) having the pressure sensors in order to form the standing surface (3), which film preferably consists of a plurality of sensor film parts, in particular of four substantially quadrantal sensor film parts, the pressure sensors of the sensor film parts preferably being connected to signal pick-ups (27) which are connected to a computing device via data transfer lines (28).

11. Posturography apparatus (1) according to any of claims 1 to 10, **characterised in that** the air cushion device (10) comprises a peripheral sealing element (10') that defines an overpressure space (11).

12. Posturography apparatus (1) according to any of claims 1 to 11, **characterised in that** the base element (9) is arranged in a frame element (29) so as to be movable in a horizontal plane, the frame element (29) preferably comprising a receiving element (13) for a guide element (12) of the base element (9), a damping stop (13') being formed between the guide element (12) and the receiving element (13).

13. Posturography apparatus (1) according to claim 12, **characterised in that** a spacer element (30) is provided between the base element (9) and the frame element (29).

14. Posturography apparatus (1) according to claim 13, **characterised in that** the spacer element (30) comprises a spring element (31) that is elastically deformable in the vertical direction.

15. Posturography apparatus (1) according to either claim 13 or claim 14, **characterised in that** the spacer element (30) comprises a sliding element (32) for sliding on a sliding surface (33) of the frame element (29).

## Revendications

1. Dispositif (1) de posturographie avec une plateforme de mesure (2) qui comporte une surface d'appui (3) destinée à un sujet à tester, avec un élément de base (9) sur lequel la plateforme de mesure (2) est supportée de façon à pouvoir basculer au moyen d'un équipement de basculement (7), et avec un équipement de coussin d'air (10) avec lequel est constitué un coussin d'air portant l'élément de base (9) et qui a pour effet une mobilité horizontale de la plateforme de mesure (2), **caractérisé en ce que**, entre la plateforme de mesure (2) et l'élément de base (9), il est disposé un élément d'amortissement (4), doté d'un matériau d'amortissement (15), avec lequel un mouvement de basculement de la plateforme de mesure (2) est amorti et suspendu.

2. Dispositif (1) de posturographie selon la revendication 1, **caractérisé en ce que** le matériau d'amortissement (15) est disposé entre la plateforme de mesure (2) et l'élément de base (9) de façon périphérique, en particulier de façon périphérique en forme d'anneau.

3. Dispositif (1) de posturographie selon la revendication 1 ou 2, **caractérisé en ce que** le matériau d'amortissement (15) est adjacent au côté inférieur de la plateforme de mesure (2) dans une position centrale essentiellement horizontale de celle-ci.

4. Dispositif (1) de posturographie selon l'une des revendications 1 à 3, **caractérisé en ce que** la section transversale du matériau d'amortissement (15) se rétrécit en direction de la plateforme de mesure (2), la section transversale du matériau d'amortissement (15) comportant une surface de délimitation (15a) essentiellement de forme parabolique.

5. Dispositif (1) de posturographie selon l'une des revendications 1 à 4, **caractérisé en ce que** le matériau d'amortissement (15) comporte une première couche (15b) du côté de la plateforme de mesure (2) et une deuxième couche (15c) du côté de l'élément de base (9), la deuxième couche (15c) étant plus dure que la première couche (15b).

6. Dispositif (1) de posturographie selon l'une des revendications 1 à 5, **caractérisé en ce qu'**une mousse, en particulier de polyuréthane, est prévue en tant que matériau d'amortissement (15).

7. Dispositif (1) de posturographie selon l'une des revendications 1 à 6, **caractérisé en ce que** l'élément d'amortissement (15) est raccordé à l'élément de base (9) par le biais d'un raccordement (16) détachable, en particulier un raccordement enfichable, et/ou **en ce que** la plateforme de mesure (2) est raccordée à l'élément d'amortissement (4) par le biais d'un autre raccordement (17) détachable, en particulier un autre raccordement enfichable.

8. Dispositif (1) de posturographie selon l'une des revendications 1 à 7, **caractérisé en ce que** l'élément d'amortissement (4) comporte au moins une ouverture de centrage (20) destinée à recevoir un élément de centrage (21) correspondant de l'élément de base (9).

9. Dispositif (1) de posturographie selon l'une des revendications 1 à 8, **caractérisé en ce que**, entre l'élément de base (9) ou l'élément d'amortissement (4) et la plateforme de mesure (2), il est prévu au moins trois éléments de levier de basculement (22) qui peuvent pivoter entre une position de libération libérant le mouvement de basculement de la plateforme de mesure (2) et une position de blocage bloquant le mouvement de basculement de la plateforme de mesure (2), des éléments d'encliquetage (25) destinés à l'encliquetage dans la position de blocage étant de préférence prévus sur les éléments de levier de basculement (22).

10. Dispositif (1) de posturographie selon l'une des revendications 1 à 9, **caractérisé en ce que** la plateforme de mesure (2) comporte une multiplicité de capteurs de pression de préférence disposés de façon essentiellement régulière sur la surface d'appui, la plateforme de mesure (2) comportant, pour la constitution de la surface d'appui (3), de préférence une feuille de capteur (26) circulaire avec les capteurs de pression, laquelle se compose de préférence de plusieurs parties de feuille de capteur, en particulier quatre parties de feuille de capteur essentiellement en forme de quart de cercle, les capteurs de pression des parties de feuille de capteur étant de préférence raccordées à des récepteurs de signaux (27) qui sont raccordés à un équipement de calcul par le biais de lignes de transfert de données (28).

11. Dispositif (1) de posturographie selon l'une des revendications 1 à 10, **caractérisé en ce que** l'équipement de coussin d'air (10) comporte un élément d'étanchéité (10') périphérique délimitant un espace de surpression (11).

12. Dispositif (1) de posturographie selon l'une des revendications 1 à 11, **caractérisé en ce que** l'élément de base (9) est disposé dans un élément de cadre (29) de façon mobile dans un plan horizontal, l'élément de cadre (29) comportant de préférence un élément de réception (13) pour un élément de guidage (12) de l'élément de base (9), une butée d'amortissement (13') étant constituée entre l'élément de guidage (12) et l'élément de réception (13).

13. Dispositif (1) de posturographie selon la revendication 12, **caractérisé en ce qu'**un élément d'espacement (30) est prévu entre l'élément de base (9) et l'élément de cadre (29).

14. Dispositif (1) de posturographie selon la revendication 13, **caractérisé en ce que** l'élément d'espacement (30) comporte un élément de ressort (31) déformable élastiquement dans la direction verticale.

15. Dispositif (1) de posturographie selon la revendication 13 ou 14, **caractérisé en ce que** l'élément d'espacement (30) comporte un élément de glissement (32) destiné au glissement sur une surface de glissement (33) de l'élément de cadre (29).
